# EUROPÄISCHE PATENTANMELDUNG

(11) **EP 1 890 150 A1**
(43) Veröffentlichungstag der Anmeldung: **20.02.2008**
(21) Anmeldenummer: 07075708.3
(22) Anmeldetag: 28.11.2003
(51) Int. Cl.: G01N 33/564, C07K 7/04, C07K 7/08, C07K 16/28, A61K 38/04, A61K 38/17, A61K 39/395

(54) **Bestimmung agonistischer, mit der humoralen Nierenabstossung assoziierten Autoantikörper**

(30) Priorität: 29.11.2002 DE 10256897; 27.01.2003 DE 10303120; 13.06.2003 DE 10327066
(62) Teilanmeldung aus: 03788849.2
(71) Anmelder: MAX-DELBRÜCK-CENTRUM FÜR MOLEKULARE MEDIZIN, 13125 Berlin (DE)
(72) Erfinder: Wallukat, Gerd, 13129 Berlin (DE)
(74) Vertreter: Boeckh, Tobias

(57) **Zusammenfassung**

Die Erfindung betrifft ein Verfahren zur Detektion von krankheitsassoziierten Autoantikörpern, die extrazelluläre Strukturen von G-Protein-gekoppelten Rezeptoren erkennen, und die Verwendung von Peptiden, die diese Loops oder Fragmente dieser umfassen, zur Behandlung von Autoimmunkrankheiten.

## Beschreibung

Die Erfindung betrifft ein Verfahren zur Detektion von krankheitsassoziierten Autoantikörpern, die Loops von G-Protein-gekoppelten Rezeptoren binden, und die Verwendung von Peptiden, die diese Loops oder Fragmente dieser umfassen, zur Behandlung der Autoimmunkrankheit humorale Nierenabstossung.

Das Immunsystem mehrzelliger Organismen beruht auf der Unterscheidung zwischen "selbst" und "nicht selbst". Aufgrund seiner großen Diversität verfügt das "selbst" und "nicht selbst" unterscheidende Immunsystem über ein großes Repertoire von Spezifitäten, die insbesondere von T- und B-Zellen exprimiert werden. Mittels komplizierter Mechanismen ist das Immunsystem zur Unterscheidung zwischen "selbst" und "nicht selbst" befähigt, mit denen sich der Körper insbesondere vor den Folgen der so genannten Autoimmunität schützen kann. Das heißt, die humoralen und zellulären Bestandteile des Immunsystems eines Organismus sind so ausgeprägt, dass sie sich nicht gegen den Organismus selbst richten. Innerhalb des Immunsystems kann es jedoch zu vielfältigen Störungen kommen, besonders können die Mechanismen der Selbstkennung eingeschränkt oder völlig ausgeschaltet werden. Daher gibt es eine Reihe von Erkrankungen, die durch Autoantikörper oder autoreaktive T-Zellen ausgelöst werden können. Eine der ersten Krankheiten, bei denen Autoantikörper gegen ein bestimmtes Organ gefunden wurden, ist die Thyreoiditis Hashimoto. Es handelt sich hierbei um eine Schilddrüsenerkrankung, die hauptsächlich bei Frauen mittleren Alters auftritt und zur Ausbildung eines Kropfes und zur Unterfunktion der Schilddrüse führt. Sofern die Krankheit nicht behandelt wird, kommt es zur vollkommenen Zerstörung und Schrumpfung des Organs. Neben Autoantikörpern, die ausschließlich gegen ein Organ gerichtet sind, gibt es zahlreiche Autoantikörper, die gegen mehrere Organe bzw. Gewebetypen gerichtet sein können.

Entsprechend der Ausrichtung der Autoantikörper können organspezifische und nichtorganspezifische Autoimmunerkrankungen unterschieden werden. Typische organspezifische Autoimmunerkrankungen sind beispielsweise die vorzeitige Menopause, der juvenile Diabetes, männliche Unfruchtbarkeit, perniziöse Anämie oder Morbus Addison. Zu den nichtorganspezifischen Autoimmunerkrankungen gehören beispielsweise die rheumatoide Arthritis, die Dermatomyositis, die Sklerodermie oder gemischte Bindegewebserkrankungen und andere. Als Zielorgane der organspezifischen Erkrankung sind häufig die Schilddrüse, die Nebenniere, der Magen und das Pankreas befallen, die nichtorganspezifischen Erkrankungen werden unter dem Begriff des so genannten rheumatischen Formenkreises zusammengefasst und betreffen die Haut, die Niere, die Gelenke und die Muskeln. Für die Diagnose und die Behandlung von Autoimmunerkrankungen sind bisher nur wenige, unzureichende Verfahren bekannt. Mit den bekannten Laborroutinen, wie beispielsweise ELISA oder anderen gut eingeführten diagnostischen Verfahren, die sich beispielsweise im Massenscreening unter den Laborbedingungen einer Klinik bewährt haben, ist es nicht möglich, die oft nur in geringen Konzentrationen vorhandenen Autoantikörper im Serum eines Patienten nachzuweisen. Die Behandlung erfolgt bei organspezifischen Erkrankungen meistens durch Herstellung des metabolischen Gleichgewichtes; zum Beispiel wird bei einer Schilddrüsenunterfunktion das fehlende Schilddrüsenhormon mit Thyroxin substituiert, und bei der Thyreotoxikose können Antimetaboliten des Hormons gegeben werden. Bei der perniziösen Anämie kann ein Depot von Vitamin B₁₂ parenteral verabreicht werden und bei der Myasthenia gravis können Cholinesterase-Inhibitoren gegeben werden. Sofern ein kompletter Funktionsverlust des Organs eingetreten ist, sind beispielsweise eine Organübertragung oder eine Implantation einer Prothese möglich. Derartige Therapieverfahren sind bei organunspezifischen Autoimmunerkrankungen nicht geeignet, da hierzu beispielsweise eine ganze Gruppe von Organen, wie beispielsweise die Haut, die Niere, die Gelenke und die Muskeln, in einem Patienten substituiert werden müsste, wobei alleine die Substitution der Gelenke, der Muskeln und/oder der Haut nahezu unmöglich ist. Eine weitere Möglichkeit der Behandlung von Autoimmunkrankheiten ist die Möglichkeit, die die Krankheit induzierenden Autoantikörper zu binden, zu komplexieren und folgend aus dem Serum zu eliminieren. Derartige Verfahren können jedoch nur dann erfolgreich in Diagnose oder Therapie eingesetzt werden, wenn ein Target, ein Agens oder eine Struktur bekannt sind, mit denen die Autoantikörper so wechselwirken, dass sie mit Hilfe des Agens oder des Targets detektiert oder eliminiert werden können. Für zahlreiche Autoimmunerkrankungen, wie zum Beispiel die Autoantikörper assoziierte Hypertonie, die Präeklampsie, die humorale Nierenabstoßung oder die Chagas Kardiomyopathie, sind derartige Agenzien nicht bekannt. Bisherige Diagnoseverfahren, wie beispielsweise Bioassays, sind schwer zu handhaben und daher für die Laborroutine ungeeignet. Bekannte Bioassays sind z. B. Herzmyozyten-Kulturen zum Nachweis von Angiotensin II AT1-Rezeptor-Autoantikörpern.

Aufgabe der Erfindung war es daher, Mittel, Vorrichtungen und Verfahren zur Diagnose von Autoantikörpern und Therapien von Autoimmunerkrankungen bereitzustellen, die eine einfache, effiziente und sichere Detektion oder Behandlung erlauben und die genannten Nachteile nicht aufweisen, wobei die Autoimmunerkrankung die humorale Nierenabstossung ist.

Die Erfindung löst dieses technische Problem durch die Bereitstellung eines Verfahrens zur Detektion von krankheitsassoziierten Autoantikörpern, die gegen G-Protein-gekoppelte Rezeptoren gerichtet und mit der humoralen Nierenabstossung assoziiert sind, wobei das Verfahren folgende Schritte umfasst:
a) In-Kontakt-Bringen von Körperflüssigkeit mit einem denaturierenden Agens,
b) In-Kontakt-Bringen der gefällten Fraktion mit einem insbesondere Biotin umfassenden Peptid, welches eine Teilsequenz des ersten und/oder zweiten Loops des Rezeptors umfasst, wobei eine Mixtur entsteht,
c) Inkubation der Mixtur mit einem insbesondere Avidin oder Streptavidin-beschichteten Träger,
d) Waschen der Materialien des Trägers,
e) Inkubation der Träger, mit anti-IgG-Subklassen, wobei der anti-IgG-Antikörper markiert ist und
f) Durchführung einer Detektions-, insbesondere einer Enzym- oder Farbreaktion.

Die Erfindung betrifft also die überraschende Lehre, dass es möglich ist, mit Hilfe einer Enzym- oder Farbreaktion, beispielsweise in einem in Laborroutinen üblichen ELISA, krankheitsassoziierte Autoantikörper, die insbesondere gegen G-Protein-gekoppelte Rezeptoren gerichtet sind, zu detektieren. Dies betrifft insbesondere Autoantikörper, der humoralen Nierenabstoßung, in Verbindung stehen, bevorzugt sind die Antikörper agonistischer Autoantikörper. Bevorzugt diese Krankheiten sind mit Hilfe eines erfindungsgemäßen enzymgekoppelten Immuntests einfach, sicherer und effektiv detektierbar. Derartige Krankheiten, insbesondere die mit ihnen assoziierten Antikörper, konnten bisher nur mit komplizierten indirekten Tests, wie zum Beispiel Bioassays, nachgewiesen werden. So wurden beispielsweise Angiotensin II-AT1-Rezeptor-Autoantikörper, die beispielsweise mit der Präeklampsie assoziiert sind, bisher sicher nur durch das In-Kontakt-Bringen der jeweiligen Seren mit kultivierten Kardiomyozyten, insbesondere neugeborener Ratten, über die Modifikation der Schlagfrequenz detektiert. Hierzu war es erforderlich, die Kardiomyozyten neugeborener Ratten zu kultivieren und diese mit dem Serum der Patienten in Kontakt zu bringen, wobei durch den Nachweis der Zunahme der Schläge pro Minute Antikörper gegen den AT1-Rezeptor bei Patienten mit Präeklampsie detektierbar waren. Mit dem erfindungsgemäßen Verfahren ist ein Immuntest zur Verfügung gestellt, mit dem zahlreiche Autoantikörper, die gegen G-Protein-gekoppelte Rezeptoren gerichtet sind, detektiert werden können. Bei diesen Autoantikörpern handelt es sich bevorzugt um einen gegen Betal-adrenergen-Rezeptor gerichteten Autoantikörper, einen gegen muskarinergen M2-Rezeptor gerichteten Autoantikörper, einen Angiotensin II AT1-Rezeptor-Autoantikörper, einen Alphal-adrenergen-Rezeptor-Autoantikörper und Autoantikörper, die gegen Endothelin IA, PAR-1, PAR-2 und/oder PAR-3 gerichtet sind. Die Autoantikörper können insbesondere agonistisch wirkende Autoantikörper sein. Selbstverständlich können die Antikörper auch inhibitorische Antikörper sein, z.B. beim allergischen Asthma (Interaktion mit dem 3. Loop). Ein insbesondere Streptavidin-beschichteter Träger ist ein Träger der bevorzugt mit Streptavidin oder Avidin beschichtet ist. Besonders bevorzugt sind aus 6 bis 2, insbesondere 4 Untereinheiten bestehende Proteine M_{R} ca. 40000 bis 80000, insbesondere 60000 oder 66000, isoelektrischen Punkt nahe dem Neutralpunkt, wie z.B. Streptavidin oder Avidin, welche eine hohe Affinität (z.B. K_{D} = 10⁻¹⁵ M⁻¹) zu anderen, insbesondere durch Cholin inaktivierende Verbindungen, bevorzugt Biotin, zeigen. Dem Fachmann ist bekannt, dass ein insbesondere Streptavidinbeschichteter Träger vorteilhaft ist, wenn eine mit diesem wirkverbindbare Struktur mit Biotin oder einem Äquivalent assoziiert ist. Wird durch Versuche ermittelt, dass die Bindung auch ohne eine Biotin/Streptavidin/Avidin Assoziierung oder Bindung für den Nachweis ausreichend ist, muss das Peptid oder der Träger nicht mit diesen Hilfsstoffen (Biotin und Streptavidin/Avidin) verbunden werden.

Das Peptid ist im Sinne der Erfindung ein Molekül, welches im Wesentlichen aus Aminosäuren besteht. Peptide im Sinne der Erfindung sind auch Strukturen, die mehr als 50 bzw. 100 Aminosäuren umfassen und somit auch als Proteine bezeichnet werden können. Peptide und Proteine werden demgemäss im Zusammenhang mit der Erfindung synonym verwendet. Selbstverständlich können die Peptide andere Strukturen wie Lipide oder Kohlenhydrate aber auch artifizielle oder natürliche Aminosäure- oder nicht-Aminosäurebausteine umfassen.

Das Biotin-umfassende Peptid oder Protein kann auch mit einem anderen Tag als Biotin kombiniert werden. Ein Tag im Sinne der Erfindung ist ein Protein-Anhängsel oder ein Peptid bzw. eine andere Struktur, die mit dem Peptid oder Protein kombiniert, beispielsweise fusioniert, wird. Das Peptid kann - wie bereits ausgeführt - beispielsweise biotinyliert vorliegen und somit Biotin als Tag aufweisen. Dem Fachmann sind weitere Tag-Strukturen oder Tags aus Katalogen und Standardwerken der Biochemie bekannt. Bevorzugte Tags sind His-Tag, Flag-Tag, Strep-Tag, T7-Tag (11 N-terminale Aminosäuren desT7Gen10-Proteins), S-Tag, CBP (Calmodulin-Binde-Peptid), MBP (Maltose-Binde-Peptid), Neb, Protein A, GST-Tag, PinPoint-Tag, Thioredoxin, PET (Cellulose-Binde-Domäne), PMal (Maltose-Binde-Domäne) und/oder Biotin-Tag. Dieses Tag besitzt eine hohe Affinität zu einer anti-Tag Substanz oder einem anti-Tag an oder auf dem Träger. Bevorzugte anti-Tags sind Streptavidin-, Glutathion-, Biotin-, Nickel-NTA-, Cellulose-, Amylose-, Thiobond-, Avidin-, und/oder Immunglobulin. Dem Fachmann sind Tag/anti-Tag Paare bekannt, d.h. die Auswahl des Tags bestimmt die Struktur des anti-Tags, ohne dass eine erfinderische Auswahl durch einen Fachmann erforderlich ist oder das dieser bei der Ausführung der erfindungsgemässen Lehre zunächst eine technische Aufgabe lösen muss. Wenn das Peptid biotinyliert vorliegt und somit Biotin als Tag aufweist, ist die anti-Tag-Struktur Streptavidin, d.h. ein mit Streptavidin beschichteter Träger. Weitere bevorzugte Tag/anti-Tag Paare sind: Protein A/ Immunglobulin, GST (Glutathion-S-Transferase)/ Glutathion, Pin-Point (in vivo Biotinylierung)/ Avidin, Thioredoxin/ Thiobond, PET (Cellulose-Binde-Domäne)/ Cellulose und/oder PMal (Maltose-Binde-Domäne)/ Amylose und andere. Bevorzugte anti-Tag-beschichtete Träger sind ausgewählt aus der Gruppe umfassend Streptavidin-, Glutathion-, Biotin-, Nickel-NTA-, Cellulose-, Amylose-, Thiobond-, Avidin- und/oder Immunglobulin-beschichtete Träger.

Die loops im Sinne der Erfindung können extrazelluläre Strukturen sein, mit denen funktionelle, agonistische oder antagonistische Autoantikörper wechselwirken, diese erkennen oder binden.

In einer besonderen Ausführungsform der Erfindung ist das denaturierende Agens Ammoniumsulfat. Selbstverständlich kann aber jedes wenig strukturverändernde Denaturierungsmittel verwendet werden, wie z.B. Alkohol bei der Alkoholfällung. Vorteilhafterweise ist es mit Ammoniumsulfat oder Alkohol möglich, Körperflüssigkeiten, wie beispielsweise Serum, zu fällen, insbesondere fraktioniert zu fällen. So können beispielsweise Antikörper, insbesondere Autoantikörper, von anderen Bestandteilen der Körperflüssigkeit separiert werden. Die Denaturierung der Körperflüssigeit erfolgt hierbei insbesondere so, dass die abgetrennten Bestandteile mittels dem Fachmann bekannter Methoden wieder in einen im Wesentlichen nativen Zustand zurückgeführt werden können oder in einen Zustand, der ihre Detektion ermöglicht. Selbstverständlich ist jedes dem Fachmann bekannte denaturierende Agens, das von Ammoniumsulfat verschieden ist, oder ein anderes, die Struktur wenig veränderndes Denaturierungsmittel, zur Fällung von Körperflüssigkeiten geeignet.

In einer weiteren bevorzugten Ausführungsform der Erfindung ist der Träger ein magnetisches Partikel oder eine ELISA-Platte bzw. eine andere Struktur, die zur Inkubation der Mixtur geeignet ist. Magnetische Partikel erlauben insbesondere die Abtrennung der gebundenen Mixtur mit Hilfe von Strom oder einer magnetischen Ladung. Eine Verwendung von ELISA-Platten ermöglicht bevorzugt den Einsatz von Laborroutinen oder standardisierten Geräten, da ELISA-Platten, insbesondere 96-well-Mikrotiterplatten, in Klinik- und Grundlagenforschung als ein Standard eingesetzt werden.

In einer weiteren bevorzugten Ausführungsform der Erfindung ist der Autoantikörper gegen einen Betal-adrenergen-Rezeptor, einen muskarinergen M2-Rezeptor, einen Angiotensin II AT1-Rezeptor, einen Alphal-adrenergen-Rezeptor, einen Endothelin IA-Rezeptor, einen PAR-1, PAR-2 und/oder PAR-3 gerichtet. Bevorzugt handelt es sich bei den Rezeptoren um G-Protein-gekoppelte Rezeptoren.

In einer besonders bevorzugten Ausführungsform der Erfindung sind, die gegen den Angiotensin II AT1-Rezeptor gerichteten Autoantikörper mit der humoralen Nierenabstoßung assoziiert. Vorteilhafterweise handelt es sich hierbei um Autoimmunerkrankungen, bei denen die krankheitsassoziierten Autoantikörper gegen bestimmte extrazelluläre Strukturen des G-Protein-gekoppelten Rezeptors gerichtet sind. Derartige Autoimmunerkrankungen sind mit bisherigen Mitteln der Laborroutine schwer oder gar nicht diagnostizierbar und weiterhin können sie nur mit erheblichem, teils chirurgischem Aufwand - z.B. Herztransplantation oder Implantation von Herzunterstützungssystemen - behandelt werden, insbesondere dadurch, dass einzelne Gewebe, Organbereiche oder vollständige Organe durch Prothesen oder andere Organe, beispielsweise von lebenden oder toten Patienten, substituiert werden.

In einer ganz besonderen Ausführungsform der Erfindung wird bei der Detektion, der humoralen Nierenabstoßung wird das Peptid eingesetzt, das eine Sequenz oder Teilsequenz des zweiten Loops des Rezeptors umfasst. Mit Vorteil ist es möglich, den ersten und/oder zweiten Loop bzw. nur den zweiten Loop bzw. Peptide, die Anteile bzw. Fragmente des ersten und/oder zweiten Loops bzw. nur des zweiten Loops umfassen, zur Detektion oder Therapie der genannten Krankheiten einzusetzen. Mit Vorteil sind dem Fachmann durch die Offenbarung des Zusammenhangs von Autoantikörper, Loop und der jeweiligen Autoimmunkrankheit verschiedene Möglichkeiten gegeben, die genannten Autoimmunkrankheiten zu diagnostizieren, prognostizieren, therapieren, sie nachzubehandeln bzw. im Verlauf einer Behandlung einer Verlaufskontrolle des jeweiligen Heilverfahrens zu unterziehen. Die Loops bzw. die Peptide, die Teilbereiche der Loops umfassen, sind bevorzugt modifiziert.

Dem Fachmann ist bekannt, dass einzelne Aminosäuren analoge physikochemische Eigenschaften aufweisen, die mit Vorteil dazu führen, dass diese Aminosäuren untereinander ausgetauscht werden können. Hierzu gehören beispielsweise die Gruppe der Aminosäuren (a) Glycin, Alanin, Valin, Leucin und/oder Isoleucin; bzw. die Aminosäuren (b) Serin und Threonin, die Aminosäuren (c) Asparagin und Glutamin, die Aminosäuren (d) Asparaginsäure und Glutaminsäure; die Aminosäuren (e) Lysin und Arginin sowie die Gruppe der aromatischen Aminosäuren (f) Phenylalanin, Tyrosin und/oder Tryptophan. Aminosäuren innerhalb ein und derselben Gruppe (a-f) können untereinander ausgetauscht werden. Weiterhin ist es möglich, dass Aminosäuren durch modifizierte Aminosäuren oder spezifische Enantiomere ausgetauscht werden. Weitere Modifikationen sind gemäß der Lehre nach der WO99/62933 oder WO02/38592 möglich.

Im Stand der Technik sind verschiedene Möglichkeiten zur Herstellung von Peptiden offenbart. Peptide, die von den erfindungsgemäßen Peptiden ausgehend mit solchen Verfahren designt werden, sind von der erfindungemäßen Lehre mit erfasst. Eine Möglichkeit des Generierens von funktionsanalogen Peptiden ist beispielsweise in PNAS USA 1998, Oct. 13; 9521:12179-84, WO 99/6293 und/oder WO 02/38592 beschrieben; diese Lehren sind in den Offenbarungsgehalt der Erfindung mit aufgenommen. Das heißt, sämtliche Peptide, Peptidfragmente oder Strukturen, die Peptide umfassen, die mit dem genannten Verfahren - von den erfindungsgemäßen Peptiden ausgehend - generiert wurden, sind Peptide im Sinne der Erfindung, sofern sie die erfindungsgemäße Aufgabe lösen, insbesondere mit den krankheitsverursachenden Autoantikörpern wechselwirken. Bei diesen Autoantikörpern kann es sich beispielsweise um agonistische Autoantikörper handeln, die Rezeptoren aktivieren oder um inhibitorische Antikörper.

In einer weiteren bevorzugten Ausführungsform der Erfindung ist es bevorzugt, dass
- die mit der humoralen Nierenabstoßung assoziierten Autoantikörper mit dem Peptid umfassend eine Sequenz oder Teilsequenz des zweiten Loops des Angiotensin II AT1-Rezeptors in Kontakt gebracht werden.

In einer weiteren bevorzugten Ausführungsform der Erfindung sind die IgG-Subklassen IgG1, IgG2, IgG3 und/oder IgG4 Subklassen. Vorteilhafterweise werden spezifische Subklassen eingesetzt, um krankheitsassoziierte Autoantikörper einfach und effektiv zu detektieren. Hierdurch ist im Gegensatz zu den Verfahren ohne die Verwendung von spezifischen Subklassen eine einfache und sichere Detektion von Autoantikörpern durchführbar. Die Subklassen können überraschenderweise bestimmten Krankheitsbildern zugeordnet werden. Es ist demgemäß im Wesentlichen kein Gemisch von IG-Sublassen mit einem Krankheitsbild assoziiert, was dahingehend überraschend ist, da im Laufe der Generierung von Subklassen durch Switch und andere biochemische Mechanismen verschiedene Subklassen generiert werden.

In einer weiteren vorteilhaften Ausführungsform ist es bevorzugt, dass
- bei der humoralen Nierenabstoßung die IgG1 und IgG3 Subklassen verwendet werden.

Mit Vorteil kann durch diese Ausgestaltungsform der Erfindung für eine spezifische Autoimmunkrankheit bzw. für die Autoantikörper eine IgG-Subklasse bzw. -Subklassen bereitgestellt werden, mit denen Autoantikörper einfach und sicher detektiert werden können.

In einer weiteren bevorzugten Ausführungsform der Erfindung werden die Autoantikörper vor dem Nachweis mit dem Fachmann bekannten Methoden aufkonzentriert oder gereinigt.

In einer besonderen Ausführungsform der Erfindung umfasst das Verfahren zum Aufkonzentrieren oder Reinigen der Autoantikörper die folgenden Schritte:
a) Gewinnung einer IgG-Fraktion aus Körperflüssigkeit,
b) In-Kontakt-Bringen der gewonnenen IgG-Fraktion mit einem Peptid, welches eine Teilsequenz eines ersten oder zweiten Loops eines G-Protein-gekoppelten Rezeptors umfasst, wobei eine Mixtur gewonnen wird,
c) Inkubation der Mixtur mit einem Träger, der gewaschen und konzentriert wird und
d) Eluieren der Autoantikörper von dem aufkonzentrierten Träger.

Zweckmäßigerweise wird durch die Reinigung oder Aufkonzentrierung eine sehr sichere Diagnose oder Detektion der Autoantikörper möglich.

In einer besonderen Ausführungsform der Erfindung ist das Peptid, welches die Sequenz bzw. eine Teilsequenz des ersten und/oder zweiten Loops umfasst, ausgewählt aus der Gruppe umfassend:
EYGSFF, SFFCEL, ARRCYND, PKCCDF, AESDE, CYIQFF, EDGECY, VRTVEDGECYIQFFSNAAVTFGTAI, AFHYESQ, ENTNIT, FWAFGR, GRAFCDV, ITEEAGY, ERFCGI, GRIFCD und/oder ITTCHDVL.

Insbesondere können diese Peptide wie folgt zugeordnet werden (siehe auch Tab. 3):
AT1 humor.
Nierenabst. II loop ENTNIT, AFHYESQ.

Es ist dem Fachmann selbstverständlich bekannt, dass er aufgrund der Offenbarung bestimmter Sequenzabschnitte des Loopes auch Sequenzen verwenden kann, die die genannten Sequenzen im natürlich vorkommenden loop flankieren. Weiterhin ist dem Fachmann bekannt, dass er die Sequenzen durch Deletionen, Substitutionen, Additionen, Insertionen oder andere biochemische bzw. biophysikalische Vorgänge so verändern kann, dass einzelne Parameter von ihnen - wie beispielsweise ihre Wirkung als diagnostisches oder therapeutisches Mittel - verbessert werden können.

In einer weiteren bevorzugten Ausführungsform der Erfindung ist insbesondere das Peptid immobilisiert. Im Sinne der Erfindung werden unter Immobilisierung verschiedene Verfahren und Techniken zum Fixieren der Peptide auf bestimmten Trägern verstanden. Die Immobilisierung kann beispielsweise der Stabilisierung der Peptide dienen, wodurch diese insbesondere bei Lagerung oder bei einmaligem Batch-Ansatz durch biologische, chemische oder physikalische Einwirkungen in ihrer Aktivität nicht reduziert oder nachteilig modifiziert werden. Durch die Immobilisierung der Peptide ist ein wiederholter Einsatz unter technischen oder klinischen Routine-Bedingungen möglich; weiterhin kann eine Probe - bevorzugt Blutbestandteile - mit mindestens einem der erfindungsgemäßen Peptide kontinuierlich umgesetzt werden. Dies kann insbesondere durch verschiedene Immobilisierungstechniken erreicht werden, wobei die Bindung der Peptide an andere Peptide oder Moleküle bzw. an einen Träger so erfolgt, dass die dreidimensionale Struktur, insbesondere an dem Zentrum, das die Wechselwirkung mit den Autoantikörpern vermittelt, der entsprechenden Moleküle, insbesondere der Peptide, nicht verändert wird. Vorteilhafterweise geht die Spezifität,zu den Autoantikörpern der Patienten durch die Immobilisierung nicht verloren. Im Sinne der Erfindung können drei grundsätzliche Methoden zur Immobilisierung verwendet werden:
(i) Quervernetzung: Bei der Quervernetzung werden die Peptide miteinander fixiert, ohne dass ihre Aktivität nachteilig beeinflusst wird. Sie sind vorteilhafterweise durch die Quervernetzung nicht mehr löslich.
(ii) Bindung an einen Träger: Die Bindung an einen Träger erfolgt zum Beispiel durch Adsorption, Ionenbindung oder kovalente Bindung. Dies kann auch innerhalb von mikrobiellen Zellen bzw. Liposomen oder anderen membranhaltigen geschlossenen bzw. offenen Strukturen erfolgen. Die Peptide werden durch die Fixierung vorteilhafterweise nicht in ihrer Aktivität beeinflusst. Die Peptide können mit Vorteil zum Beispiel in der Klinik in Diagnose oder Therapie trägergebunden mehrfach oder kontinuierlich eingesetzt werden.
(iii) Einschluss: Der Einschluss erfolgt im Sinne der Erfindung insbesondere an eine semipermeable Membran in Form von Gelen, Fibrillen oder Fasern. Gekapselte Peptide sind durch eine semipermeable Membran so durch die umgebende Probenlösung getrennt, dass sie vorteilhafterweise noch mit den Autoantikörpern oder mit Fragmenten dieser interagieren können. Für die Immobilisierung stehen verschiedene Verfahren zur Verfügung, wie beispielsweise die Adsorption an einen inerten oder elektrisch geladenen anorganischen oder organischen Träger. Solche Träger können beispielsweise poröse Gele, Aluminiumoxid, Betonid, Agarose, Stärke, Nylon oder Polyacrylamid sein. Die Immobilisierung erfolgt hierbei durch physikalische Bindungskräfte, oft unter Beteiligung von hydrophoben Wechselwirkungen und ionischen Bindungen. Derartige Methoden sind vorteilhafterweise einfach zu handhaben und sie beeinflussen die Konformation der Peptide nur in geringem Umfang. Durch elektrostatische Bindungskräfte zwischen den geladenen Gruppen der Peptide und dem Träger kann die Bindung vorteilhafterweise verbessert werden, zum Beispiel durch die Verwendung von Ionenaustauschern, insbesondere Sephadex.

Ein weiteres Verfahren ist die kovalente Bindung an Trägermaterialien. Die Träger können dazu reaktive Gruppen aufweisen, die mit Aminosäure-Seitenketten homöopolare Bindungen eingehen. Geeignete Gruppen in Peptiden sind Carboxy-, Hydroxy- und Sulfidgruppen und insbesondere die endständigen Aminogruppen von Lysinen. Aromatische Gruppen bieten die Möglichkeit für Diazo-Kopplungen. Die Oberfläche von mikroskopischen porösen Glaspartikeln kann durch Behandlung mit Silanen aktiviert und anschließend mit Peptiden umgesetzt werden. Hydroxy-Gruppen natürlicher Polymere können zum Beispiel mit Bromzyan aktiviert und anschließend mit Peptiden gekoppelt werden. Mit Polyacrylamid-Harzen können zahlreiche Peptide vorteilhafterweise direkte kovalente Bindungen eingehen. Bei dem Einschluss in dreidimensionale Netzwerke werden die Peptide in ionotrophe Gele oder andere dem Fachmann bekannte Strukturen eingeschlossen. Die Poren der Matrix sind insbesondere so beschaffen, dass die Peptide zurückgehalten werden und eine Interaktion mit den Ziel-Molekülen möglich ist. Bei der Quervernetzung werden die Peptide durch Vernetzung mit bifunktionellen Agenzien in polymere Aggregate umgewandelt. Derartige Strukturen sind gelatinös und leicht verformbar und insbesondere für den Einsatz in verschiedenen Reaktoren geeignet. Durch Zugabe anderer inaktiver Komponenten, wie zum Beispiel Gelatine, bei der Vernetzung können die mechanischen und Bindungseigenschaften vorteilhafterweise verbessert werden. Bei der Mikroverkapselung wird der Reaktionsraum der Peptide mit Hilfe von Membranen eingegrenzt. Die Mikroverkapselung kann zum Beispiel als Grenzflächen-Polymerisation durchgeführt werden. Durch die Immobilisierung bei der Mikroverkapselung werden die Peptide unlöslich und dadurch wieder verwendbar. Im Sinne der Erfindung sind immobilisierte Peptide alle Peptide, die sich in einem Zustand befinden, der ihre Wiederverwendung erlaubt. Die Einschränkung der Beweglichkeit und der Löslichkeit der Peptide auf chemischem, biologischem oder physikalischem Wege führt vorteilhafterweise zu niedrigen Verfahrenskosten, insbesondere bei der Eliminierung von Autoantikörpern aus Blutbestandteilen.

In einer weiteren bevorzugten Ausführungsform der Erfindung ist das Peptid an eine Festphase gebunden. Die Bindung des Peptides an die Festphase kann über einen Spacer erfolgen. Als Spacer können alle chemischen Verbindungen eingesetzt werden, die für die Funktion des Spacers die geeigneten strukturellen und funktionellen Voraussetzungen aufweisen, solange sie nicht das Bindeverhalten derart modifizieren, dass eine Bindung des Autoantikörpers mit dem Peptid nachteilhafterweise beeinträchtigt wird.

In einer besonders bevorzugten Ausführungsform der Erfindung umfasst das Peptid Aminogruppen, Amide, Acetylgruppen, Biotingruppen, Marker, Spacer, Linker, GKK und/oder SGKK. Derartige Strukturen erlauben vorteilhafterweise eine Verwendung der Peptide in der Apheresetherapie.

In einer besonders bevorzugten Ausführungsform der Erfindung umfasst der Linker und/oder der Spacer α-Aminocarbonsäuren sowie deren Homo- und Heterooligomere; α,ω-Aminocarbonsäuren sowie deren verzweigte Homo- oder Heterooligomere; sonstige Aminosäuren sowie die linearen und verzweigten Homo- oder Heterooligomere; Aminooligoalkoxy-alkylamine; Maleinimidocarbonsäure-Derivate; Oligomere von Alkylaminen; 4-Alkylphenyl-Derivate; 4-Oligoalkoxyphenyl- oder 4-Oligoalkoxyphenoxy-Derivate; 4-Oligoalkylmercaptophenyl- oder 4-Oligoalkylmercaptophenoxy-Derivate; 4-Oligoalkylaminphenyl- oder 4-Oligoalkylaminyphenoxy-Derivate; (Oligoalkylbenzyl)-phenyl- oder 4-Oligoalkylbenzyl)-phenoxy-Derivate sowie 4-Oligoalkoxybenzyl)-phenyl- oder 4-Oligoalkoxybenzyl)-phenoxy-Derivate; Trityl-Derivate; Benzyloxyaryl- oder Benzyloxyalkyl-Derivate; Xanthen-3-yl-oxyalkyl-Derivate; (4-Alkylphenyl)- oder ω-(4-Alkylphenoxy)-alkansäure-Derivate; Oligoalkyl-Phenoxyalkyl- oder Oligoalkoxy-phenoxyalkyl-Derivate; Carbamat-Derivate; Amine; Trialkylsilyl- oder Dialkyl-alkoxysilyl-Derivate; Alkyl- oder Aryl-Derivate und/oder Kombinationen davon.

In einer weiteren besonders bevorzugten Ausführungsform der Erfindung sind die immobilisierten Peptide durch Deletion, Addition, Substitution, Translokation, Inversion und/oder Insertion modifiziert.

Die Erfindung betrifft auch ein Peptid ausgewählt aus der Gruppe umfassend EYGSFF und/oder SFFCEL (DCM, 1. loop); ARRCYND und/oder PKCCDF (DCM, 2. loop); AESDE (Chagas, 2. loop), CYIQFF und/oder EDGECY (DCM, 2. loop); VRTVEDGECYIQFFSNAAVTFGTAI (Chagas, 2. loop), AFHYESQ (Präeklampsie, 2. loop), ENTNIT und/oder AFHYESQ (humorale Nienabstossung, maligne Hypertonie, 2. loop); FWAFGR und/oder GRAFCDV (essentielle Hypertonie, 1. loop), ITEEAGY und/oder ERFCGI (essentielle Hypertonie, 2. loop), GRIFCD, GRAFCDV (Psariasis, 1. loop) und/oder ITTCHDVL zur Verwendung als medizinischer Wirkstoff. Bei der pulmonaren und refraktären Hypertonie gelten zum 1. und 2. loop die Ausführungen zur essentiellen Hypertonie. Die Verwendung als therapeutischer Wirkstoff meint im Sinne der Erfindung die Anwendung des Peptids bzw. der Peptide auf dem gesamten Gebiet der Medizin, bevorzugt zur Diagnose und Therapie von Autoimmunkrankheiten.

Dem Fachmann ist bekannt, dass er aufgrund der offenbarten Peptide als Diagnose- und/oder Therapiemittel weitere funktionsanaloge Peptide generieren kann. Diese funktionsanalogen Peptide sind durch die erfindungsgemäße Lehre mit erfasst. Insbesondere ist auf die Dissertationen PNAS USA 1998, Oct. 13; 9521:12179-84, WO 99/6293 und/oder WO 02/38592 hingewiesen, die in den Offenbarungsgehalt der erfindungsgemäßen Lehre mit aufgenommen sind.

In einer bevorzugten Ausführungsform der Erfindung wird das Peptid von Autoantikörpern von Patienten mit einer der folgenden Krankheiten gebunden: humorale Nierenabstoßung. Der Fachmann kann aus dieser Offenbarung durch Routineversuche Diagnose- und Therapieverfahren bereitstellen.

Die Erfindung betrifft auch Erkennungsmoleküle, die gegen das erfindungsgemäße Peptid gerichtet sind. Bevorzugt sind die Erkennungsmoleküle Antikörper, Antisense-Konstrukte und/oder ein Chelatoren. Die erfindungsgemäßen Erkennungsmoleküle können Antikörper sein, die gegen Autoantikörper gerichtet sind, die insbesondere folgende Krankheiten induzieren: die humorale Nierenabstoßung.

Die Erfindung betrifft auch eine pharmazeutische Zusammensetzung, die die Peptide und/oder die Erkennungsmoleküle gegebenenfalls mit einem pharmazeutisch verträglichen Träger umfasst. Die pharmazeutische Zusammensetzung kann insbesondere als Arzneimittel eingesetzt werden. Hierzu ist es beispielsweise möglich, die Peptide durch Zyklisierung oder andere dem Fachmann bekannte Verfahren so zu modifizieren, dass sie durch körpereigene peptidabbauende Strukturen, wie zum Beispiel Serumproteasen, nicht zerstört werden können. Durch Verwendung der erfindungsgemäßen Peptide oder Erkennungsmoleküle ist es möglich, die Autoantikörper in oder ex vivo bzw. in vitro zu neutralisieren. Eine in-vitro Neutralisation ist beispielsweise bei der Untersuchung von Autoimmunerkrankungen in Gewebe- oder Zellkulturen vorteilhaft. Bei einer in vivo Neutralisation werden die Arzneimittel dem Patienten direkt verabreicht, bei einer ex vivo Neutralisation wird beispielsweise das Blut über eine Schleife - zum Beispiel in Form eines Schlauch-Kreislaufes - aus dem Körper geleitet, folgend mit dem Arzneimittel in Kontakt gebracht und nach der erfolgten Neutralisation der Autoantikörper wieder in den Organismus, insbesondere dem humanen Patienten, zurückgeführt. Im Sinne der Erfindung gelten als Arzneimittel sowohl solche pharmazeutischen Zusammensetzungen, die für die therapeutischen und prophylaktischen Zwecke verwendet werden als auch solche pharmazeutischen Zusammensetzungen, die als Diagnostikum eingesetzt werden können.

Arzneimittel oder pharmazeutische Zusammensetzungen, die vorliegend synonym verwendet werden, sind erfindungsgemäß Stoffe und Zubereitungen aus Stoffen, die dazu bestimmt sind, durch Anwendung am oder im menschlichen Körper Krankheiten, Leiden, Körperschäden oder krankhafte Beschwerden zu heilen, zu lindern oder zu verhüten. Medizinische Hilfsstoffe sind erfindungsgemäß solche Stoffe, die zur Produktion als aktive Ingredienzien von Arzneimitteln eingesetzt werden. Pharmazeutisch-technische Hilfsstoffe dienen der geeigneten Formulierung des Arzneimittels oder der pharmazeutischen Zusammensetzung und können sogar, sofern sie nur während des Herstellungsverfahrens benötigt werden, anschließend entfernt werden oder können als pharmazeutisch verträgliche Träger Teil der pharmazeutischen Zusammensetzung sein. Die Arzneimittelformulierung oder Formulierung der pharmazeutischen Zusammensetzung erfolgt gegebenenfalls in Kombination mit einem pharmazeutisch verträglichen Träger und/oder Verdünnungsmittel. Beispiele für geeignete pharmazeutisch verträgliche Träger sind dem Fachmann bekannt und umfassen zum Beispiel Phosphatgepufferte Kochsalzlösungen, Wasser, Emulsionen wie zum Beispiel Öl/Wasser-Emulsionen, verschiedene Arten von Detergenzien, sterile Lösungen etc., Arzneimittel oder pharmazeutische Zusammensetzungen, die solche Träger umfassen, können mittels bekannter konventioneller Methoden formuliert werden. Diese Arzneimittel oder pharmazeutischen Zusammensetzungen können einem Individuum in einer geeigneten Dosis verabreicht werden, beispielsweise in einem Bereich von 1 µg bis 10 g an Peptiden pro Tag und Patient. Bevorzugt werden dabei Dosen von 1 mg bis 1 g. Bevorzugt wird eine Verabreichung von möglichst wenigen und niedrigen Dosen und weiter bevorzugt eine einmalige Dosis. Die Verabreichung kann auf verschiedenen Wegen erfolgen, beispielsweise intravenös, intraperitoneal, intrarektal, intragastrointestinal, intranodal, intramuskulär, lokal, aber auch subkutan, intradermal oder auf der Haut oder über die Schleimhäute. Die Verabreichung von Nukleinsäuren, die für das erfindungsgemäße Peptid codieren, kann auch in Form von Gen-Therapien geschehen, beispielsweise über virale Vektoren. Die Art der Dosierung und des Verabreichungsweges kann vom behandelnden Arzt entsprechend den klinischen Faktoren bestimmt werden. Es ist dem Fachmann bekannt, dass die Art der Dosierung von verschiedenen Faktoren abhängig ist, wie zum Beispiel der Größe, der Körperoberfläche, dem Alter, dem Geschlecht oder der allgemeinen Gesundheit des Patienten, aber auch von dem speziellen Mittel, welches verabreicht wird, der Dauer und Art der Verabreichung und von anderen Medikamenten, die möglicherweise parallel verabreicht werden. Der Fachmann kann sich hierbei an den üblichen Standardwerten sowie an speziellen Lehren orientieren, zum Beispiel an der Lehre der EP 1 085 955, die in den Offenbarungsgehalt der Erfindung mit aufgenommen ist. Weiterhin ist dem Fachmann bekannt, dass er die Konzentration der Autoantikörper mit den erfindungsgemäßen Peptiden zunächst diagnostizieren kann, um die notwendige Konzentration des Arzneimittels zu bestimmen.

Die pharmazeutischen Zusammensetzungen oder das Arzneimittel umfassen insbesondere eine pharmakologische Substanz, die ein oder mehrere erfindungsgemäße Peptide oder Erkennungsmoleküle oder/und diese codierende Nukleinsäuremoleküle in einer geeigneten Lösung oder Verabreichungsform enthält. Diese können entweder alleine mit den entsprechenden unter Arzneimitteln oder pharmazeutischen Zusammensetzungen beschriebenen Hilfsstoffen oder in Kombination mit einem oder mehreren Adjuvantien, beispielsweise QS-21, GPI-0100 oder andere Saponine, Wasser-Öl Emulsionen wie beispielsweise Montanide, Adjuvantien, Polylysin, Polyargininverbindungen, DNA-Verbindungen wie beispielsweise CpG, Detox, bakterielle Vakzine wie beispielsweise Thyphusvakzine oder BCG-Vakzine, Salze wie beispielsweise Kalziumphosphate und/oder einem anderen geeigneten Stoff zur Wirkungsverstärkung verabreicht werden; vorzugsweise immunstimulatorische Moleküle, wie Interleukine, beispielsweise IL-2, IL-12, IL-4 und/oder Wachstumsfaktoren, beispielsweise GM-CSF. Diese werden in bekannten Methoden mit den erfindungsgemäßen Peptiden oder Erkennungsmolekülen gemischt und in einer geeigneten Formulierung und Dosierung verabreicht. Formulierungen, Dosierungen und geeignete Komponenten sind dem Fachmann bekannt.

Die pharmazeutische Zusammensetzung oder das Arzneimittel können selbstverständlich auch eine Kombination von zwei oder mehreren der erfindungsgemäßen pharmazeutischen Zusammensetzungen oder Arzneimittel sein, sowie eine Kombination mit anderen Arzneimitteln, wie beispielsweise Antikörpertherapien, Chemotherapien oder Radiotherapien, die auf eine geeignete Weise zeitlich gemeinsam oder getrennt verabreicht bzw. angewandt werden. Die Herstellung der Arzneimittel oder pharmazeutischen Zusammensetzungen erfolgt nach an sich bekannten Methoden.

Die Erfindung betrifft auch einen Kit, der das erfindungsgemäße Peptid, die erfindungsgemäßen Erkennungsmoleküle und/oder die erfindungsgemäße pharmazeutische Zusammensetzung umfasst, gegebenenfalls mit einer Anweisung zum Kombinieren der Inhalte des Kits und/oder zum Bereitstellen einer Formulierung für einen Empfänger und einem Algorithmus der Gabe der Formulierung, das heißt in welcher Dosis oder in welchen Zeitintervallen einem Patienten einzelne Bestandteile des Kits verabreicht werden. Der Empfänger im Sinne der Erfindung kann jedoch auch eine Zelle oder ein Gewebe in vivo, ex vivo oder in vitro sein. Bei der Information kann es sich beispielsweise um einen Beipackzettel, aber auch um eine Information handeln, die der Anwender fernmündlich oder über Internet abrufen kann. Der Algorithmus der Gabe der Formulierung beinhaltet insbesondere eine Anleitung zum diagnostischen und/oder therapeutischen Verfahren zur Behandlung eines Patienten. Hierbei kann es sich um einstufige als auch mehrstufige Verfahren handeln, als auch um solche, die in Ab- oder Anwesenheit eines Arztes durchgeführt werden. Das heißt, das Therapieschema bzw. die Information über dieses sind bevorzugt ein Bestandteil des Kits.

Die Erfindung betrifft auch eine Vorrichtung zur Chromatographie, die die erfindungsgemäßen Peptide umfasst.

In einer bevorzugten Ausführungsform sind die Peptide innerhalb des Chromatographiesystems beispielsweise an einer Festphase gebunden.

Die erfindungsgemäße Vorrichtung kann insbesondere dazu verwendet werden, die Autoantikörper aus Flüssigkeiten eines Patienten zu eliminieren bzw. die Autoantikörper zu neutralisieren. Dieses Verfahren ist dem Fachmann unter dem Begriff der Immunadsorption oder Apheresetherapie bekannt. Mit Hilfe der Immunadsorption werden Immunglobuline aus dem Blut des Patienten entfernt. Vorteilhafterweise kann diese Immunadsorptionsbehandlung stationär und ambulant durchgeführt werden. Es kann vorgesehen sein, dass die Vorrichtung, insbesondere der so genannte Adsorber, Bestandteil eines extrakorporalen Blutkreislaufes ist. Hierbei wird dem Patienten aus einem größeren Körpergefäß, insbesondere einer Armvene, kontinuierlich bzw. diskontinuierlich Blut entnommen und mittels Filtration oder Zentrifugation in einzelne Bestandteile, wie beispielsweise die zellulären und die humoralen Bestandteile, separiert. Ein wesentlicher Bestandteil des Blutes, das hierdurch gewonnen wird, ist insbesondere Blutplasma. Das Blutplasma kann vorteilhafterweise durch die erfindungsgemäße Vorrichtung geleitet und nach Adsorption der Autoantikörper zusammen mit den zuvor separierten Blutbestandteilen, insbesondere den zellulären Bestandteilen, dem Patienten zurückgegeben werden, insbesondere durch eine andere Arm- bzw. Beinvene. Es kann weiterhin vorgesehen sein, dass die Peptide an einer Sepharose-Matrix immobilisiert sind. Diese Matrix kann in einen Behälter gegeben werden, der ein Volumen von 10 bis 400 ml aufweist. Das Blutplasma des Patienten kann dann über diese Matrix geleitet werden, wobei die Autoantikörper binden und so aus dem Blutplasma eliminiert werden können. Dem Fachmann sind verschiedene Möglichkeiten bekannt, derartige festphasenfixierte Peptide bereitzustellen, beispielsweise in Form von (i) regenerationsfähigen adsorptionssäulen, in Form von (ii) Doppelsäulen als auch in Form von (iii) Einmalsäulen. Die verschiedenen Spül- und Elutionslösungen, die eine hohe Effizienz der Behandlung ermöglichen, können durch den Fachmann problemlos durch Routineversuche ermittelt werden. Durch die Bereitstellung der erfindungsgemäßen Lehre, insbesondere der erfindungsgemäßen Peptide, sind dem Fachmann verschiedene Möglichkeiten offenbart, diese in vivo, ex vivo und in vitro, zur Prophylaxe, Diagnose, Therapie als auch zur Nachbehandlung der dilatativen Kardiomyopathie, der Chagas Kardiomyopathie, der Myokarditis, der Präeklampsie, der humoralen Nierenabstoßung, der malignen Hypertonie, der essentiellen Hypertonie, der refraktären Hypertonie, der pulmonaren Hypertonie, der Psoriasis und/oder des Raynaud-Syndroms einzusetzen. Dem Fachmann sind weitere Ausgestaltungen aus der WO 02/38592, der EP 1 214 350 und der WO 99/56126 bekannt, die in den Offenbarungsgehalt der erfindungsgemäßen Lehre mit aufgenommen sind.

Die Erfindung betrifft auch die Verwendung der erfindungsgemäßen Peptide, der erfindungsgemäßen pharmazeutischen Zusammensetzung, des erfindungsgemäßen Kits und/oder der erfindungsgemäßen Vorrichtung zur Prophylaxe, Diagnose, Therapie, Verlaufskontrolle und/oder Nachbehandlung von Autoimmunkrankheiten ausgewählt aus der Gruppe umfassend die humorale Nierenabstoßung.

Die Erfindung betrifft auch die Verwendung der erfindungsgemäßen Peptide, der erfindungsgemäßen pharmazeutischen Zusammensetzung, des erfindungsgemäßen Kits und/oder der erfindungsgemäßen Vorrichtung zur Herstellung eines Arzneimittels zur Behandlung von Autoimmunkrankheiten ausgewählt aus der Gruppe umfassend die humorale Nierenabstoßsung.

Die Erfindung betrifft auch die Verwendung der erfindungsgemäßen Peptide, der erfindungsgemäßen pharmazeutischen Zusammensetzung, des erfindungsgemäßen Kits und/oder der erfindungsgemäßen Vorrichtung zum Screenen von Arzneimitteln. Das Screenen von Arzneimitteln kann beispielsweise die Identifizierung von Substanzen, insbesondere Peptiden, Proteinen, Kohlenhydraten und/oder Lipiden umfassen, die mit den Peptiden wechselwirken. Eine Wechselwirkung kann beispielsweise eine Bindung an diese Peptide sein oder aber auch eine Aktivierung bzw. Inhibierung von oder durch die genannten Peptide. Ein Arzneimittel könnte demgemäß beispielsweise eine Struktur sein, die im Körper eines Patienten an die Peptide, und dementsprechend an die entsprechenden Loops bindet und so mit den dort vorkommenden Autoantikörpern um eine Bindungsstelle konkurriert. Durch die Offenbarung der erfindungsgemäßen Lehre, insbesondere über die Offenbarung des Zusammenhangs von Krankheit und dem Bindungsort der Autoantikörper, kann der Fachmann verschiedene Arzneimittel screenen. Das Screenen von Arzneimitteln aufgrund von offenbarten Targets gehört zum allgemeinen Wissen des Fachmanns und erfolgt durch Routineversuche, es sei auf die entsprechenden Standardwerke der Molekularbiologie und Pharmakologie verwiesen.

Die Erfindung betrifft auch ein Verfahren zur Behandlung einer Autoimmunkrankheit ausgewählt aus der Gruppe umfassend, die humorale Nierenabstoßung, durch eine Bindung und/oder Entfernung von Autoantikörpern mittels von an eine Festphase gebundenen erfindungsgemäßen Peptiden. Durch die an die Festphase gebundenen Peptide werden die Autoantikörper gebunden, komplexiert und/oder an der Festphase neutralisiert.

In einer besonderen Ausführungsform des Behandlungsverfahrens ist bevorzugt, dass die Autoantikörper bei der humoralen Nierenabstoßung gegen Angiotensin II AT1-Rezeptoren, gerichtet sind.

Im Folgenden soll die Erfindung anhand eines Beispiels näher erläutert werden, ohne auf dieses Beispiel beschränkt zu sein.

### Beispiel

### Bestimmung von Angiotensin II-AT₁-Rezeptor-Autoantikörpern

Spontan schlagende, kultivierte Kardiomyozyten neugeborener Ratten sind ein sehr nützliches Modell für die Untersuchung der Wirkung von Autoantikörpern.

Über Forschungen an β₁-Adrenorezeptor-Autoantikörpern wurde bereits von Wallukat et al., 2001, berichtet. Dieser Bericht handelt von Angiotensin II-AT₁-Rezeptor-Autoantikörpern bei präeklamptischen Frauen. Präeklampsie ist eine Störung, die sich durch eine Zunahme des Blutdrucks zu erkennen gibt, der zum Tod von Mutter und Fötus führen kann. Dechend et al., 2000, waren in der Lage, die Manifestation agonistischer Antikörper gegen Angiotensin AT₁-Rezeptoren aufzuzeigen, die bei präeklamptischen Frauen häufig auftreten. Mit der Aktivierung des AT₁-Rezeptors durch agonistische Autoantikörper ließen sich viele der pathophysiologischen Merkmale der Präeklampsie erklären. Die Befunde von Wallukat et al., 1999, zeigen, dass Immunglobulin-Fraktionen und affinitätsgereinigte Antikörper von präeklamptischen Frauen den AT₁-Rezeptor von kultivierten Kardiomyozyten stimulieren können. Durch Zugabe von Losartan (1 µM) verringern sich die Schläge pro Minute. Durch Neutralisationsexperimente konnte gezeigt werden, dass die IgG-Unterklasse 3 für die Zunahme der Schlagfrequenz verantwortlich ist.

Gemäß diesen Befunden wurde ein enzymgekoppelter Immuntest zur Bestimmung von Angiotensin II-AT₁-Rezeptor-Autoantikörpern (Anti-AT₁-AAB) entwickelt.

Erstens: Peptid-Lösungen, entsprechend der Aminosäuresequenz der zweiten Schleife des menschlichen AT₁-Rezeptors (Sm 1986/1, 100 µg/ml), wurden mit Anti-AT₁-AAB (1:1; Vol./Vol.) 1 Stunde lang bei 4 °C inkubiert. Anti-AT₁-AABs wurden hergestellt durch Ammoniumsulfat-Fällung aus Abfallflüssigkeiten bei der Geburt (Blut und isotonische Salzlösung). Diese Proben waren stärker konzentriert als Reinserumproben.

Zweitens: Diese Mischung wurde mit gewaschenen Streptavidin-beschichteten magnetischen Teilchen 1 Stunde lang bei 4 °C inkubiert.

Drittens: Zur Abtrennung der IgG/Peptid-Mischung wurden die magnetischen Teilchen dreimal mit Waschpuffer gewaschen (20 mM Kaliumphosphat-Puffer, 0,15 M NaCl, pH 7,5). Das Abtrennen oder Waschen kann ohne weiteres mit einem Magnet-Konzentrationsapparat (Dynal) durchgeführt werden. Unspezifische Bindungsstellen wurden mit 1 % Rinderserumalbumin in Waschpuffer blockiert.

Viertens: Die magnetischen Teilchen wurden mit einer Lösung von Meerettichperoxidase-markierten Antikörpern gegen menschliches IgG3 inkubiert (1:200, 1 Stunde, Raumtemperatur).

Fünftens: Die Teilchen wurden mit einer standardisierten gebrauchsfertigen Lösung von TMB (Tetramethylbenzidin) 30 min lang bei Raumtemperatur im Dunkeln behandelt. Die Farbreaktionen (blau-grün) wurden mit 0,1 N HCl gestoppt (gelb-orange). Die optischen Dichten wurden in einem Mikroplatten-Lesegerät (Anthos HTII) bei 492 nm (Bezugsfilter 620 nm) gemessen. Die Ergebnisse sind in Tabelle 1 zusammengefasst.

Das gleiche Peptid des menschlichen AT₁-Rezeptors (Sm 1986/1) wurde zur Reinigung von Anti-AT₁-AABs verwendet. IgG-Lösungen wurden mit Peptid-Lösung (100 µg/ml, 1:1; Vol./Vol.) gemischt und eine Stunde lang bei 4 °C inkubiert. Die dreimal gewaschenen, Streptavidin-beschichteten magnetischen Teilchen wurden zugegeben (300 µl). Die Teilchen wurden mit dem Magnet-Konzentrationsapparat eingesammelt. Die Überstände wurden vorsichtig abgetrennt und in Eis gelagert. Die magnetischen Teilchen wurden dreimal gewaschen und mit 3 M Kaliumthiocyanat-Lösung 15 min lang bei Raumtemperatur eluiert. Nach magnetischer Aufkonzentrierung wurden die Lösungen vorsichtig abgetrennt und zusammen mit dem ersten Überstand gegen NaCl (0,9 %) in Phosphat-gepufferter Lösung dialysiert. Nach 5-maligem Austausch innerhalb von 3 Tagen wurde der Proteingehalt anhand der optischen Dichte bestimmt (280 nm). Die chronotrope Wirkung von Überstand und Eluat auf primäre kultivierte Kardiomyozyten neugeborener Ratten (Bioassay) wurde mit einem bildgebenden Computersystem (IMAGOQANT) aufgezeichnet.

Tabelle 2 zeigt die Reproduzierbarkeit des Reinigungsverfahrens. Sechs der sechs gereinigten Anti-AT₁-AABs zeigten die Zunahme der Schlagfrequenz/min (>24,4). Mit Überstand behandelte Kulturen bewirkten dagegen keine oder nur mäßige Änderungen der Schlagfrequenz (<10,0).

Das Verfahren der Coimmunpräzipitation des AT₁-Rezeptors war ähnlich der Methode des β₁-Adrenorezeptors (Wallukat, 2001). Die Unterschiede sind: es wurden lysierte Membranen transfizierter CHO-Zellen (Couchon, 1997) für die Coimmunpräzipitation verwendet. Die lysierten Membranen sollten frisch hergestellt werden. Die Proteine wurden identifiziert mit Hilfe eines Antikörpers gegen ein Peptid mit der Sequenz des N-terminalen Teils des AT₁-Rezeptors, der in Kaninchen erzeugt worden war (N10, 1 :100, Santa Cruz), und durch Western-Blot und ECL-System mit Anti-Kaninchen-IgG-Peroxidase-Konjugaten (1 : 10 000, Sigma) nachgewiesen.

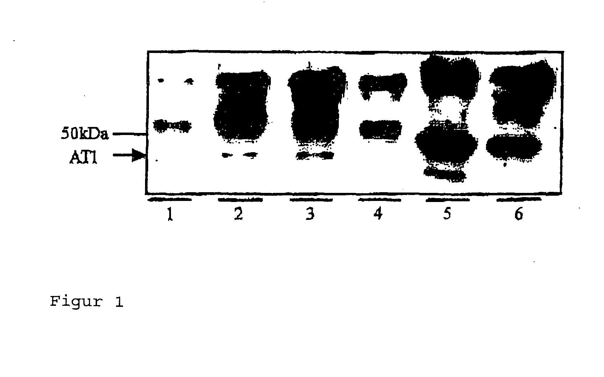

Figur 1 zeigt die Ergebnisse des Western-Blots. Eine Bande (Molekulargewicht >40,0 kDa) konnte mit Hilfe interner positiver Proben (lysierte Membranen transfizierter CHO-Zellen und menschliches Plazenta-Gewebe) exakt erfasst werden. Bei früheren Experimenten (Neichel, unveröffentlichte Daten) konnte diese Bande durch die Peptide blockiert werden, die zur Herstellung der N10-Antikörper verwendet wurden. Diese Bande fehlte in reinen Sepharose-Proben und in den Überständen der Reinigungsexperimente.

Die Ergebnisse zeigen die Nützlichkeit des bildgebenden Computersystems IMAGOQUANT beim Nachweisen der Zunahme der Schläge/min durch AT₁-AABs bei Patientinnen mit Präeklampsie. Der enzymgekoppelte Immuntest sollte mit Seren von Präeklampsie-Patientinnen und gesunden Spendern geprüft werden. Die gereinigten AT₁-AABs können zur weiteren Untersuchung der Pathogenese der Präeklampsie verwendet werden.

**Tabelle 1**

| Messung der AT₁-Autoantikörper mit Hilfe eines enzymgekoppelten Immuntests | | |
|---|---|---|
| IgG | n | Optische Dichte (OD, 492 nm) Bereich |
| | | |
| Gesunde (Kontrollen) | 3 | 0,036 - 0,069 |
| Präeklamptische Frau | | |
| positiv | 15 | 0,071 - 0,786 |
| negativ | 4 | 0,021 - 0,069 |

**Tabelle 2**

| Einfluss von Überständen und Eluaten der magnetischen Teilchen auf die Schlagfrequenz kultivierter Kardiomyozyten neugeborener Ratten | | | | | | |
|---|---|---|---|---|---|---|
| Patientin/ Tag des Experiments | Proben | OD | µg/ml | Bioassay (Zunahme der Schläge/min) | | |
| | | | | 1:100 | 1:50 | 1:20 |
| D. 19.03.02 | Überstand | 4,300 | 3071,4 | 6,0±0,0 | 6,0±0,0 | 10,0±1,6 |
| | Eluat | 0,086 | 61,4 | 12,8±1,6 | 27,6±2,0 | 34,4±1,2 |
| D. 27.05.02 | Überstand | 6,820 | 4871,4 | -1,6±0,8 | 4,0±1,2 | 6,4±1,2 |
| | Eluat | 0,033 | 23,6 | 12,1±2,4 | 18,9±0,5 | 24,5±0,8 |
| D. 03.06.02 | Überstand | | | 3,3±0,8 | 3,2±0,8 | 4,7±1,6 |
| | Eluat | 0,0104 | 74,3 | 11,1±1.2 | 15,2±2,0 | 33,9±2,0 |

| | | | | | | |
|---|---|---|---|---|---|---|
| ± SA vom Mittelwert | | | | | | |

**Tabelle 3**

| Autoantikörper gegen G-Protein-gekoppelte Rezeptoren | | | |
|---|---|---|---|
| Angaben zu den Epitopen und IgG Subklasse | | | |
| **Antikörper gegen Rez.** | **Erkrankung** | **Epitop** | **IgGSubklasse** |
| Ang. II AT1 | humorale Nierenabstoßung | 2. loop | IgG1 u. IgG3 |

### Legende

Figur 1: Western-Blot der Coimmunpräzipitation des Angiotensin AT₁-Rezeptors
   Bahn 1 Protein A/Sepharose; 2 präeklamptische Patientin D. ohne Reinigung; 3 KSCN-Eluat; 4 Überstand; 5 lysierte CHO-Membranen; 6 lysiertes Plazenta-Gewebe.

## Patentansprüche

1. Verfahren zur Detektion von krankheitsassoziierten Autoantikörpern, die gegen G-Protein-gekoppelte Rezeptoren gerichtet und mit der humoralen Nierenabstossung assoziiert sind,
**dadurch gekennzeichnet, dass**
das Verfahren folgende Schritte umfasst:
a) In-Kontakt-Bringen von Körperflüssigkeiten mit einem denaturierenden Agens,
b) In-Kontakt-Bringen der gefällten Fraktion mit einem Biotin umfassenden Peptid, welches eine Sequenz oder Teilsequenz eines ersten und/oder zweiten Loops des Rezeptors umfasst, wobei eine Mixtur entsteht,
c) Inkubation der Mixtur mit einem Avidin oder Streptavidin-beschichteten Träger,
d) Waschen der Materialien des Trägers,
e) Inkubation der Träger mit anti-IgG-Antikörper-Subklassen, wobei der anti-IgG-Antikörper markiert ist und
f) Durchführung einer Enzym- oder Farbreaktion.

2. Verfahren nach Anspruch 1,
**dadurch gekennzeichnet, dass**
das denaturierende Agens Ammoniumsulfat und/oder Alkohol ist.

3. Verfahren nach Anspruch 1,
**dadurch gekennzeichnet, dass**
der Träger ein magnetisches Partikel oder eine ELI-SA-Platte ist.

4. Verfahren nach Anspruch 1 oder 2,
**dadurch gekennzeichnet, dass**
die Autoantikörper gegen einen Betal-adrenergen-Rezeptor, einen muskarinergen M2-Rezeptor, einen Angiotensin II AT1-Rezeptor, einen Alphal-adrenergen-Rezeptor, einen Endothelin IA-Rezeptor, einen PAR-1, PAR-2 und/oder PAR-3 gerichtet sind.

5. Verfahren nach einem der Ansprüche 1 bis 4,
**dadurch gekennzeichnet, dass**
die gegen den Angiotensin II AT1-Rezeptor gerichteten Autoantikörper mit der humoralen Nierenabstoßung assoziiert sind.

6. Verfahren nach einem der vorhergehenden Ansprüche,
**dadurch gekennzeichnet, dass**
bei der Detektion der humoralen Nierenabstoßung das Peptid eingesetzt wird, das eine Sequenz oder Teilsequenz des zweiten Loops umfasst.

7. Verfahren nach einem der vorhergehenden Ansprüche,
**dadurch gekennzeichnet, dass**
die mit der humoralen Nierenabstoßung assoziierten Autoantikörper mit dem Peptid umfassend eine Sequenz oder Teilsequenz des zweiten Loops des Angiotensin II AT1-Rezeptors in Kontakt gebracht werden.

8. Verfahren nach einem der vorhergehenden Ansprüche,
**dadurch gekennzeichnet, dass**
die IgG-Subklassen IgG1, IgG2, IgG3 und/oder IgG4 Subklassen sind.

9. Verfahren nach einem der vorhergehenden Ansprüche,
**dadurch gekennzeichnet, dass**
bei der humoralen Nierenabstoßung die IgG1 und IgG3 Subklasse verwendet wird.

10. Verfahren nach einem der vorhergehenden Ansprüche,
**dadurch gekennzeichnet, dass**
die Autoantikörper vor dem Nachweis aufkonzentriert oder gereinigt werden.

11. Verfahren nach einem der vorhergehenden Ansprüche,
**dadurch gekennzeichnet, dass**
das Peptid welches die Sequenz oder Teilsequenzen des ersten und/oder zweiten Loops umfasst, ausgewählt ist aus der Gruppe umfassend EYGSFF, SFFCEL, ARRCYND, PKCCDF, AESDE, CYIQFF, EDGECY, VRTVEDGECYIQFFSNAAVTFGTAI, AFHYESQ, ENTNIT, FWAFGR, GRAFCDV, ITEEAGY, ERFCGI, GRIFCD und/oder ITTCHDVL.

12. Peptid ausgewählt aus der Gruppe umfassend EYGSFF, SFFCEL, ARRCYND, PKCCDF, AESDE, CYIQFF, EDGECY, VRTVEDGECYIQFFSNAAVTFGTAI, AFHYESQ, ENTNIT, FWAFGR, GRAFCDV, ITEEAGY, ERFCGI, GRIFCD und ITTCHDVL zur Verwendung als medizinischer Wirkstoff.

13. Pharmazeutische Zusammensetzung, umfassend ein Peptid gemäß Anspruch 12.

14. Chromatographievorrichtung, umfassend Peptide gemäß Anspruch 12.

15. Verwendung der Peptide nach Anspruch 12 und/oder einer pharmazeutischen Zusammensetzung nach Anspruch 13 und/oder einer Vorrichtung nach Anspruch 14 zum Screenen von Arzneimitteln.

16. Verwendung der Peptide nach Anspruch 12,
**dadurch gekennzeichnet, dass**
gegen Betal-adrenergen-Rezeptor, muskarinergen M2-Rezeptor, Angeotensin II AT1-Rezeptor, AlphaIadrenergen-Rezeptor, Endothelin IA, PAR-1, PAR-2 und/oder PAR-3 gerichtete Autoantikörper detektiert, gebunden, komplexiert und/oder neutralisiert werden.

17. Verfahren zur Behandlung einer Autoimmunkrankheit, ausgewählt aus der Gruppe umfassend die humorale Nierenabstoßung durch eine Bindung und/oder Entfernung von Autoantikörpern mittels von an eine Festphase gebundenen Peptiden nach Anspruch 12.

18. Verfahren nach dem vorhergehenden Anspruch,
**dadurch gekennzeichnet, dass**
die Autoantikörper bei der humoralen Nierenabstoßung gegen Angiotensin II AT1-Rezeptoren gerichtet sind.
